# EUROPEAN PATENT SPECIFICATION

(11) **EP 2 394 593 B1**
(45) Date of publication and mention of the grant of the patent: **12.02.2014**
(21) Application number: 10826483.9
(22) Date of filing: 05.10.2010
(51) Int. Cl.: A61B 18/12, A61B 18/14

(54) **HIGH-FREQUENCY SURGERY DEVICE**
HOCHFREQUENZCHIRURGIEVORRICHTUNG
DISPOSITIF CHIRURGICAL HAUTE FRÉQUENCE

(30) Priority: 28.10.2009 US 255536 P
(43) Date of publication of application: 14.12.2011
(73) Proprietor: OLYMPUS MEDICAL SYSTEMS CORP., Tokyo 151-0072 (JP)
(72) Inventor: KABAYA, Akinori, Tokyo 151-0072 (JP); IRISAWA, Takashi, Tokyo 151-0072 (JP)
(74) Representative: Gunzelmann, Rainer
(86) International application number: PCT/JP2010/067439
(87) International publication number: WO 2011/052349

(56) References cited:
- EP-A1- 1 810 630
- EP-A1- 2 106 762
- JP-A- 8 098 845
- JP-A- 10 286 261
- JP-A- 2002 065 691
- JP-A- 2005 000 224
- JP-A- 2008 114 042
- JP-A- 2008 510 507
- US-A- 5 643 257
- US-A1- 2002 052 599
- US-A1- 2003 158 551
- US-A1- 2008 103 495

## Description

### Technical Field

The present invention relates to a high frequency surgery apparatus and a surgery control method for performing surgery by passing a high frequency current through a living tissue.

### Background Art

In recent years, various types of surgery apparatus are used in surgery and the like. For example, a technique of injecting high frequency energy into a blood vessel to perform treatment is conventionally known. In this case, a high frequency surgery apparatus is used which passes a high frequency current through the blood vessel which is being grasped with an appropriate grasping force and seals the blood vessel using thermal energy thereby generated.

For example, a high frequency surgery apparatus described in Japanese Patent Application Laid-Open Publication No.2002-325772 measures an electric impedance of a living tissue while supplying a high frequency current to the living tissue, performs control so as to sequentially reduce the output value of high frequency power in three stages, stops the output when a predetermined electric impedance is reached and ends the processing.

However, according to the method in which the output is stopped when the predetermined electric impedance is reached as described in the above conventional example, it would be difficult to readily perform surgery in a case of performing surgery (or treatment) of sealing with respect to blood vessels different in thickness as target living tissues of the surgery.

Document EP 1 810 630 A1 concerns a system and a method for performing electro surgical procedures. The system includes an electrical generator adapted to supply energy at an output level to a tissue. The electro surgical generator includes a micro processor adapted to generate a desired impedance trajectory having at least one slope.

The present invention has been made in view of the above-described point and an object of the present invention is to provide a high frequency surgery apparatus and a surgery control method capable of performing surgery such as sealing even in a case of performing the surgery on different target living tissues.

### Disclosure of Invention

### Means for Solving the Problem

A high frequency surgery apparatus according to an aspect of the present invention includes the features of claim 1.

### Brief Description of the Drawings

Fig. 1 is a diagram illustrating an overall configuration of a high frequency surgery apparatus according to a first embodiment of the present invention;
Fig. 2 is a block diagram illustrating an internal configuration of a high frequency power supply apparatus of the high frequency surgery apparatus;
Fig. 3 is a flowchart illustrating a typical example of high frequency surgery control method for a blood vessel to be treated according to the first embodiment;
Fig. 4A is an explanatory operation diagram illustrating an impedance variation when sealing treatment is applied to a large diameter blood vessel according to the high frequency surgery control method in Fig. 3 through intermittent output;
Fig. 4B is an explanatory operation diagram illustrating an impedance variation when sealing treatment is applied to a small diameter blood vessel according to the high frequency surgery control method in Fig. 3 through intermittent output;
Fig. 5A is an explanatory operation diagram illustrating an impedance variation when sealing treatment is applied to a large diameter blood vessel according to the high frequency surgery control method in Fig. 3 through continuous outputs;
Fig. 5B is an explanatory operation diagram illustrating an impedance variation when sealing treatment is applied to a small diameter blood vessel according to the high frequency surgery control method in Fig. 3 through continuous outputs;
Fig. 6A is a diagram illustrating an impedance variation when a high frequency current is supplied under the same condition to apply sealing treatment to a small diameter blood vessel and a large diameter blood vessel;
Fig. 6B is a diagram illustrating the way to realize high sealing performance by setting two control parameters according to the first embodiment;
Fig. 6C is a diagram illustrating measured data of average blood vessel withstand pressure values when sealing treatment is applied to a large diameter blood vessel and a small diameter blood vessel using an output time threshold and an impedance threshold as control parameters respectively;
Fig. 7A is a diagram illustrating measured data to determine an impedance threshold as a control parameter in the case of a large diameter blood vessel;
Fig. 7B is a diagram illustrating measured data to determine an output time threshold as a control parameter in the case of a small diameter blood vessel;
Fig. 7C is a diagram illustrating measured data to determine an output time threshold as a control parameter in the case of a medium diameter blood vessel;
Fig. 8A is a diagram illustrating constant power control and constant voltage control when performing output control according to a second embodiment of the present invention;
Fig. 8B is a flowchart illustrating a typical example of a high frequency surgery control method for a blood vessel to be treated according to the second embodiment;
Fig. 9A is an explanatory operation diagram illustrating an impedance variation or the like when sealing treatment is applied to a large diameter blood vessel according to the high frequency surgery control method of the second embodiment;
Fig. 9B is an explanatory operation diagram illustrating an impedance variation or the like when sealing treatment is applied to a small diameter blood vessel according to the high frequency surgery control method of the second embodiment;
Fig. 10 is a block diagram illustrating an internal configuration of a high frequency power supply apparatus according to a third embodiment of the present invention;
Fig. 11 is a flowchart illustrating a processing procedure for exercising output control when performing sealing treatment according to the third embodiment;
Fig. 12 is a diagram illustrating an example of measured data of an impedance variation in the case of a sample when a near-best blood vessel withstand pressure value is obtained and a sample of a near-minimum blood vessel withstand pressure value; and
Fig. 13 is a flowchart illustrating a processing procedure when performing sealing treatment in a modification example of the third embodiment.

### Best Mode for Carrying Out the Invention

Hereinafter, embodiments of the present invention will be described with reference to the accompanying drawings.

### (First embodiment)

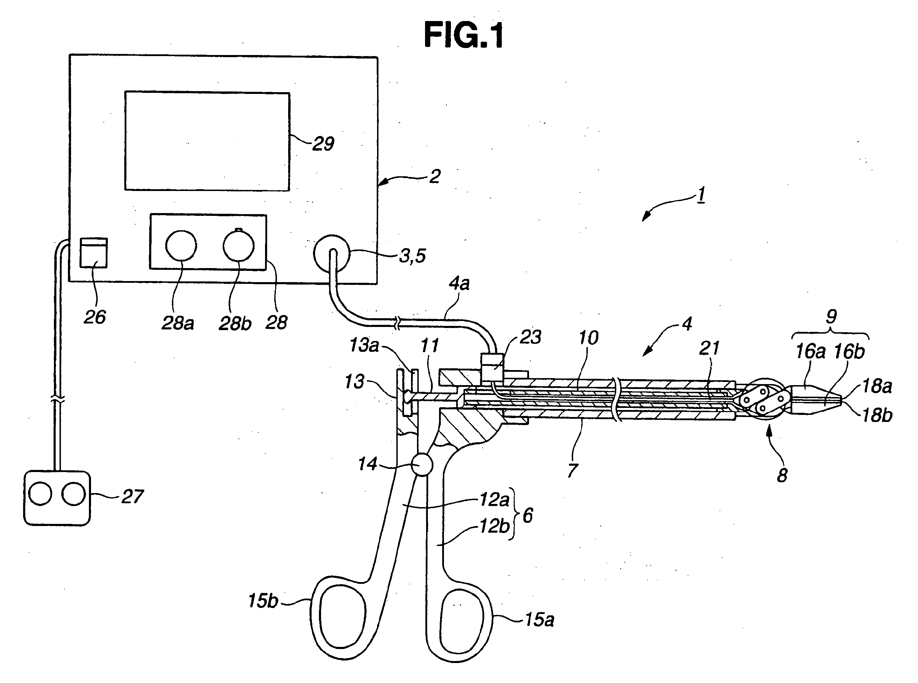

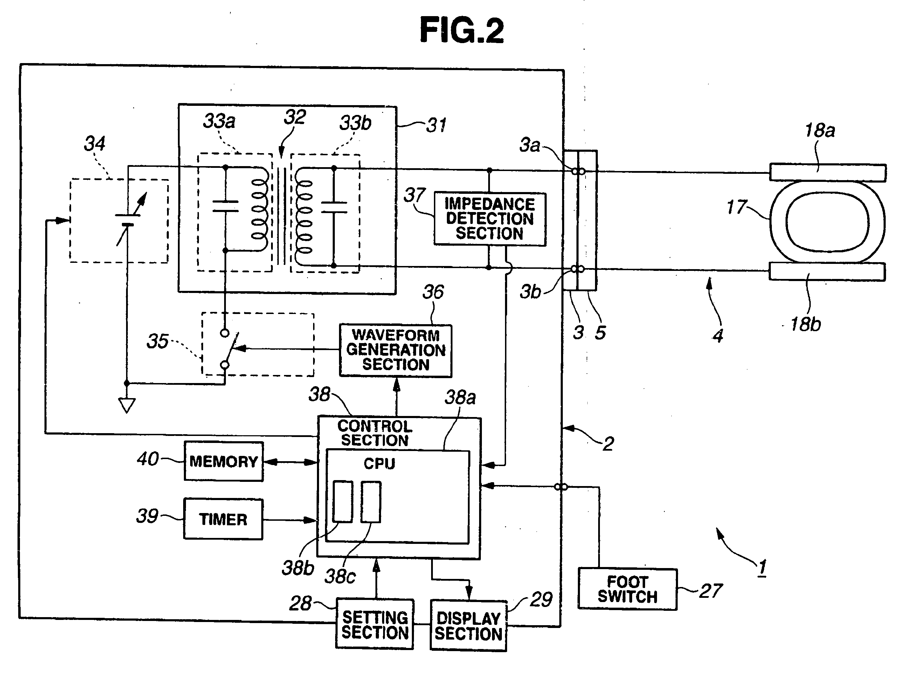

As shown in Fig. 1, a high frequency surgery apparatus 1 according to a first embodiment of the present invention includes a high frequency power supply apparatus 2 provided with a high frequency current generation section 31 that generates a high frequency current for treatment (see Fig. 2).

The high frequency power supply apparatus 2 is provided with a connector receiver 3 that outputs a high frequency current generated and a connector 5 provided at a proximal end of a connection cable 4a of a high frequency probe 4 is detachably connected to the connector receiver 3 as a high frequency treatment instrument.

The high frequency probe 4 includes an operation section 6 for an operator to grasp to operate, a sheath 7 that extends from a top end of the operation section 6 and a treatment section 9 provided via a link mechanism 8 at a distal end of the sheath 7 to pass a high frequency current through a living tissue to be treated and perform treatment of high frequency surgery.

A slide pipe 10 is inserted into the sheath 7 and a rear end of the slide pipe 10 is connected to a connection bearing 13 at one top end of handles 12a and 12b forming the operation section 6 via a connection shaft 11. The connection bearing 13 is provided with a slit 13a that allows a rear end side of the connection shaft 11 to pass and does not allow its spherical portion at the rear end to pass.

The handles 12a and 12b are pivotably coupled at a pivoted section 14 and are provided with finger hooking members 15a and 15b on the bottom end side.

When the operator performs operation of opening or closing the finger hooking members 15a and 15b, the top ends of the handles 12a and 12b move in opposite directions. The operator can then push forward or move backward the slide pipe 10.

A distal end of the slide pipe 10 is connected to a pair of treatment members 16a and 16b making up the treatment section 9 via a link mechanism 8 for opening/closing.

Therefore, the operator performs operation of opening/closing the handles 12a and 12b, and can thereby drive the link mechanism 8 connected to the slide pipe 10 that moves forward/backward and open/close the pair of treatment members 16a and 16b. The blood vessel 17 as the living tissue to be treated can be grasped using the two mutually facing inner surface parts of the pair of treatment members 16a and 16b that open/close (see Fig. 2).

The state in Fig. 1 is a state in which the handles 12a and 12b are closed and if the handles 12a and 12b are opened from this condition, the slide pipe 10 moves forward and the pair of treatment members 16a and 16b can be opened via the link mechanism 8.

The pair of treatment members 16a and 16b are provided with bipolar electrodes 18a and 18b on the inner surfaces facing each other. The rear end sides of the treatment members 16a and 16b are connected to the link mechanism 8.

A pair of signal lines 21 are passed through the slide pipe 10 and connected to the electrodes 18a and 18b respectively. Furthermore, the rear end of the signal line 21 is connected to a connector receiver 23 provided, for example, at a top of the handle 12b. A connector at the other end of the connection cable 4a is detachably connected to the connector receiver 23.

A foot switch 27 as an output switch that performs operation of instructing output ON (energization) or output OFF (disconnection) of a high frequency current is connected to the high frequency power supply apparatus 2, in addition to a power supply switch 26. The operator can step on the foot switch 27 with the foot to thereby supply or stop supplying the high frequency current to the treatment section 9.

Furthermore, a setting section 28 for setting a high frequency power value or the like is provided on the front of the high frequency power supply apparatus 2. The setting section 28 is provided with a power setting button 28a that sets a high frequency power value and a selection switch 28b that selects one of an intermittent output mode in which a high frequency current is outputted intermittently and a continuous output mode in which a high frequency current is outputted continuously. The operator is allowed to set a high frequency power value suitable for treatment and set an output mode used to perform high frequency surgery.

A display section 29 that displays the set high frequency power value or the like is provided above the setting section 28.

As shown in Fig. 2, the high frequency power supply apparatus 2 is configured by a high frequency current generation section 31 that generates a high frequency current to be transmitted to a living tissue to be operated on using an insulation transformer 32. A parallel resonance circuit 33a to which a capacitor is connected in parallel is provided on a primary wiring side of the insulation transformer 32. A DC voltage is applied to one end of the parallel resonance circuit 33a from a variable power supply 34 and a switching circuit 35 is connected to the other end thereof.

The variable power supply 34 can change and output the DC voltage. Furthermore, the switching circuit 35 performs switching through application of a switching control signal from a waveform generation section 36.

The switching circuit 35 switches a current that flows from the variable power supply 34 to the primary wiring of the insulation transformer 32 and generates a voltage-boosted high frequency current at an output section 33b on a secondary wiring side of the insulation transformer 32 insulated from the primary wiring side. A capacitor is also connected to the secondary wiring.

The output section 33b on the secondary wiring side of the insulation transformer 32 is connected to contacts 3a and 3b of the connector receiver 3 which is an output end of the high frequency current. Treatment such as sealing can be performed by transmitting a high frequency current via the high frequency probe 4 connected to the connector receiver 3 and supplying (applying) the high frequency current to a blood vessel 17 as a living tissue to be operated on.

Furthermore, both ends of the output section 33b are connected to an impedance detection section 37. The impedance detection section 37 detects a voltage between output ends (two contacts 3a and 3b) when the high frequency current is passed through the blood vessel 17 as the living tissue as shown in Fig. 2 and a current that flows through the blood vessel 17 which becomes a load and detects an electric impedance (simply abbreviated as "impedance") obtained by dividing the voltage in that case by the current. The impedance detection section 37 outputs the detected impedance to a control section 38. As will be described later, the impedance detection section 37 may also be configured so as to further calculate an impedance Za of the blood vessel 17 portion and output the impedance Za to the control section 38.

Furthermore, the control section 38 is connected to a timer 39 as a time measuring section that measures time, a memory 40 that stores various kinds of information, the foot switch 27 that turns ON or OFF the output of a high frequency current, the setting section 28 and the display section 29.

The control section 38 that controls the sections of the high frequency power supply apparatus 2 sends setting conditions and control signals corresponding to the impedance detected by the impedance detection section 37 and the measured time by the timer 39 to the variable power supply 34 and the waveform generation section 36.

The variable power supply 34 outputs DC power corresponding to the control signal sent from the control section 38. Furthermore, the waveform generation section 36 outputs a waveform (here, square wave) corresponding to the control signal sent from the control section 38.

The high frequency current generation section 31 generates a high frequency current through the operation of the switching circuit 35, which is turned ON or OFF by the DC power sent from the variable power supply 34 and the square wave sent from the waveform generation section 36 and outputs the high frequency current from the connector receiver 3. The parallel resonance circuit 33a reduces spurious caused by the square wave obtained through the switching operation. The output section 33b also forms a resonance circuit and reduces spurious.

The control section 38 is constructed, for example, of a CPU 38a and the CPU 38a controls the respective sections when performing treatment such as sealing on the blood vessel 17 according to the program stored in the memory 40.

In the present embodiment, in order to be able to appropriately perform sealing treatment on any blood vessel 17 of small to large diameter, the memory 40 stores a first threshold Tm of output time and a second threshold Zs of impedance as control parameters for appropriately performing sealing treatment.

In order to detect impedance at the connector receiver 3 to which the connector 5 at the proximal end of the high frequency probe 4 is connected, the impedance detection section 37 actually detects a net impedance Za of the blood vessel 17 at the electrodes 18a and 18b as an impedance Za' including an impedance component of the high frequency probe 4.

The present embodiment will describe that the impedance detection section 37 further calculates the net impedance Za from the impedance Za' and outputs the impedance Za to the CPU 38a. This processing may also be performed by the CPU 38a. Hereinafter, suppose the impedance detection section 37 calculates (detects) the net impedance Za of the blood vessel 17 at the electrodes 18a and 18b and outputs the net impedance Za to the CPU 38a.

The impedance threshold Zs stored in the memory 40 is a threshold set for the net impedance of the blood vessel 17 at the electrodes 18a and 18b.

When the threshold Zs' itself that corresponds to the impedance Za' detected through the measurement by the impedance detection section 37 is used instead of the threshold Zs, the impedance Za' may be compared with the threshold Zs'.

As will be described below, upon starting treatment with high frequency energy, the CPU 38a of the control section 38 has the function of the judging section 38b that measures an output time Ta via the timer 39, judges whether or not the output time Ta has reached the threshold Tm and judges whether or not the impedance Za detected by the impedance detection section 37 has reached the second threshold Zs.

Upon judging that the condition of having reached the first threshold Tm and the condition of having reached the second threshold Zs are satisfied, the CPU 38a has the function of the output control section 38c that performs output control of stopping the output of the high frequency current from the high frequency current generation section 31.

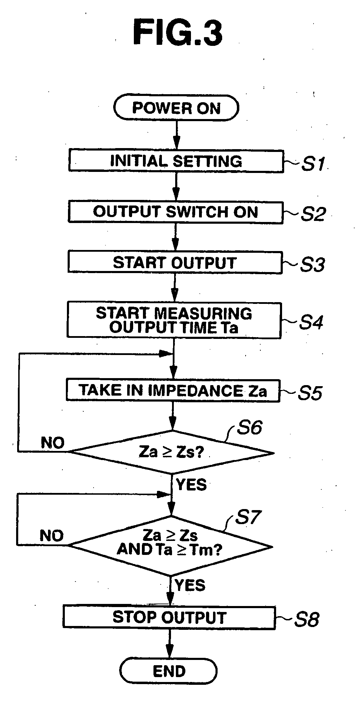

Next, the operation when performing treatment of sealing the blood vessel 17 using the high frequency probe 4 according to the present embodiment will be described with reference to a flowchart in Fig. 3.

The operator turns ON the power supply switch 26 and makes an initial setting of a high frequency power value and an output mode or the like when performing treatment as shown in step S1.

Furthermore, the operator grasps the blood vessel 17 as a living tissue to be treated using the electrodes 18a and 18b of the treatment section 9 at the distal end portion of the high frequency probe 4 shown in Fig. 1. Fig. 2 schematically shows the blood vessel 17 as the living tissue grasped by the electrodes 18a and 18b.

As shown in step S2, the operator turns ON the foot switch 27 as an output switch to perform sealing treatment on the blood vessel 17. The output switch may also be provided in the high frequency probe 4.

When the output switch is turned ON, the CPU 38 of the control section 38 controls the high frequency current generation section 31 so as to generate a high frequency current. The high frequency current generation section 31 outputs the high frequency current from the output end and the high frequency probe 4 transmits the high frequency current and supplies the high frequency current to the blood vessel 17 contacting the electrodes 18a and 18b. The high frequency current flows through the blood vessel 17 and sealing treatment starts. That is, the output of the high frequency current in step S3 in Fig. 3 starts.

At this moment, as shown in step S4, the CPU 38a causes the timer 39 as the time measuring section to start measurement (counting) of the output time Ta of the high frequency current.

Furthermore, as shown in step S5, the CPU 38a takes in the impedance Za detected (measured) by the impedance detection section 37 in a predetermined cycle.

As shown in next step S6, the CPU 38a judges whether or not the impedance Za taken in has reached a preset second threshold Zs, that is, Za≥Zs.

When the condition of Za≥Zs is not satisfied (that is, Za<Zs), the CPU 38a returns to the processing in step S5.

On the other hand, when the judgment result shows that the condition of Za≥Zs is satisfied, the CPU 38a moves to processing in step S7. In step S7, the CPU 38a judges whether or not the output time Ta measured by the timer 39 has reached the first threshold Tm, that is, judges whether or not Ta≥Tm. When the CPU 38a performs judgment in step S7, since the judgment in step S6 has already proved that the condition of Za≥Zs is satisfied, step S7 is processing of substantially judging whether or not Za≥Zs and Ta≥Tm.

When the judgment result in step S7 does not satisfy Ta≥Tm (that is, Ta<Tm), the CPU 38a returns to the processing in step S7. On the other hand, when the judgment result shows that the condition of Ta≥Tm is satisfied, the CPU 38a moves to the processing in step S8. In step S8, the CPU 38a performs control of stopping the output. The CPU 38a then ends the control processing on the sealing treatment in Fig. 3.

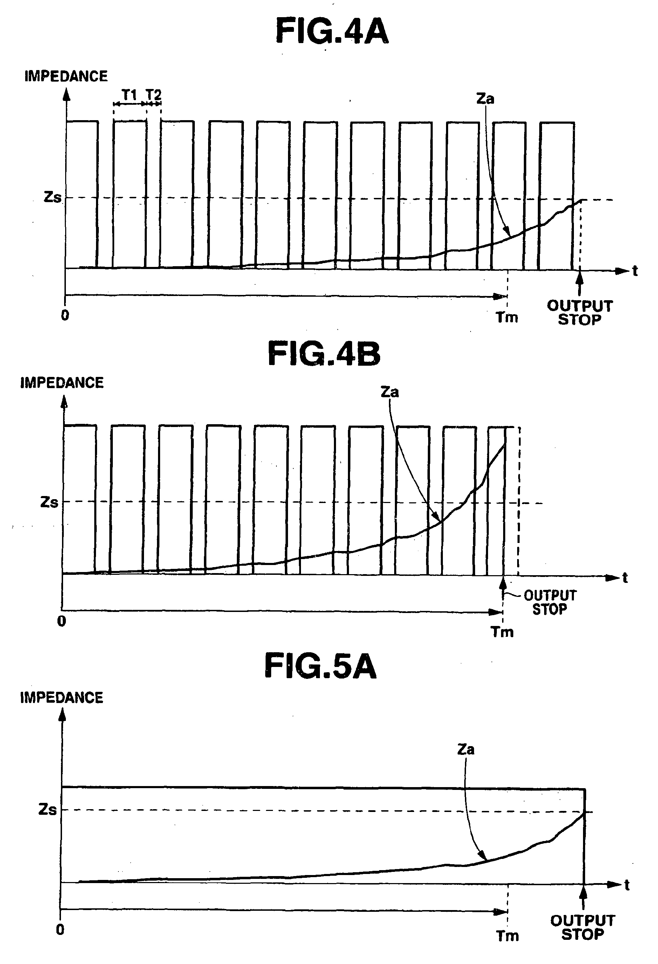

Fig. 4A illustrates a typical variation of the impedance Za when the high frequency current is set to an intermittent output mode and sealing treatment is applied to a large diameter blood vessel. Here, the horizontal axis shows time t and the vertical axis shows an impedance. Fig. 4A (the same applies to Fig. 4B or the like) also illustrates a situation in which a high frequency current is intermittently outputted in the intermittent output mode.

In the case of the intermittent output mode, the present embodiment has such a setting that a first period T1 for outputting a high frequency current intermittently and a second period T2 for stopping the output, the first period T1 and the second period T2 forming a cycle, are set to 2:1. The periods T1 and T2 are set to 60 ms and 30 ms respectively. Furthermore, during the period in this intermittent output mode, the high frequency current is set to a constant power value.

A typical variation of the impedance Za when sealing treatment is applied to a small diameter blood vessel under output conditions similar to those in the case with Fig. 4A is as shown in Fig. 4B.

As is clear from Fig. 4A and Fig. 4B, when treatment is applied to the large diameter blood vessel, the value of impedance Za increases relatively slowly. The impedance Za is smaller than the second threshold Zs even when the output time Ta reaches the first threshold Tm.

Thus, the intermittent output mode continues even when the time exceeds the first threshold Tm. The output is stopped when the impedance Za reaches (exceeds) the second threshold Zs.

On the other hand, in the case of the treatment on the small diameter blood vessel, compared to the case with the large diameter blood vessel, the value of impedance Za increases earlier. The impedance Za exceeds the second threshold Zs before the output time Ta reaches the first threshold Tm.

When the intermittent output mode continues with the value of impedance Za exceeding the second threshold Zs and the output time Ta reaches (exceeds) the first threshold Tm, the output is stopped. In Fig. 4B, if the intermittent output is stopped at timing at which the output time Ta exceeds the first threshold Tm, the output may also be stopped at timing slightly delayed as shown by a dotted line.

Although Fig. 4A and Fig. 4B illustrate a case where sealing treatment is applied to in the intermittent output mode, treatment may also be performed in a continuous output mode.

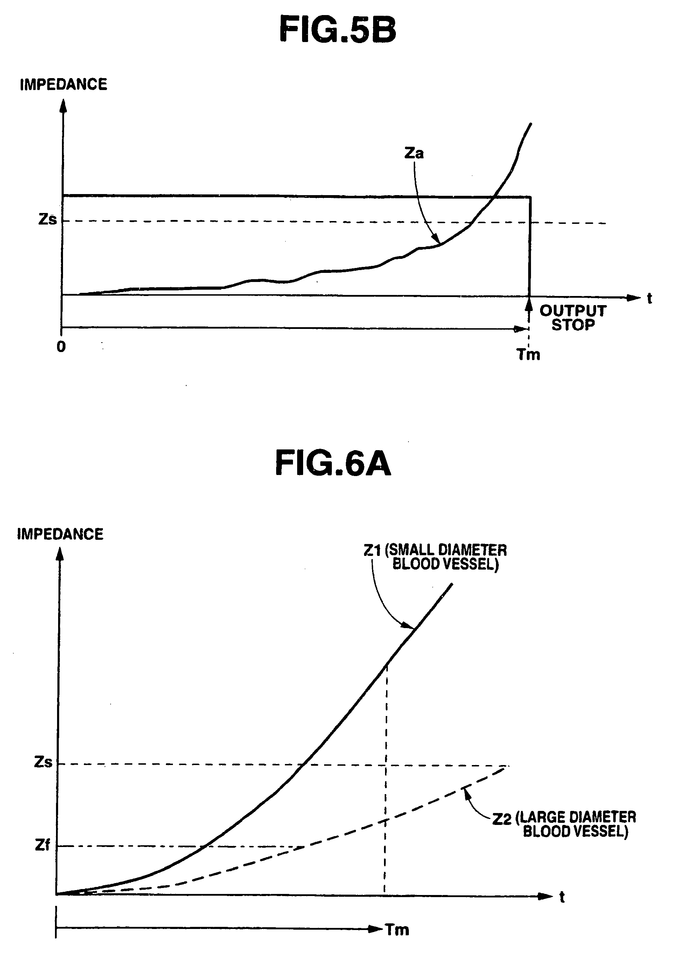

Fig. 5A and Fig. 5B illustrate a typical variation of impedance Za when sealing treatment is applied to a large diameter blood vessel and a small diameter blood vessel in the continuous output mode.

The tendency (situation) of variation of impedance Za when treatment is performed in the continuous output mode is similar to that in the case described in Fig. 4A and Fig. 4B.

As described above, the present embodiment sets the first threshold Tm corresponding to the output time Ta and the second threshold Zs corresponding to the value of impedance Za, performs sealing treatment with a high frequency current, and can thereby appropriately perform sealing treatment on the blood vessel 17 of small (to be more specific, on the order of 1 mm) to large diameter (to be more specific, on the order of 7 mm).

Thus, the operator can smoothly perform sealing treatment on the blood vessel 17 and the burden on the operator when performing sealing treatment can be alleviated. Furthermore, since sealing treatment can be performed smoothly, the surgery time can be reduced.

The effectiveness in performing such control according to the present invention will be described below. As is clear from characteristics of variation of impedance Za in Fig. 4A to Fig. 5B, in the case of a small diameter blood vessel, the value of impedance Za increases together with the output time Ta in a shorter time than in the case of a large diameter blood vessel.

A common sealing mechanism includes concrescence and coagulation. In the case of a small diameter blood vessel, sealing can be realized through coagulation by dehydration of removing water content, but in the case of a large diameter blood vessel, sealing is realized using concrescence whereby mainly collagen in the blood vessel is heated and liquefied.

Thus, in the case of the small diameter blood vessel, sealing characteristics do not deteriorate even when the treatment time extends, whereas sealing characteristics are affected in the case of the large diameter blood vessel.

A solid line and a dotted line in Fig. 6A schematically indicate variations of impedances Z1 and Z2 of the small diameter blood vessel and the large diameter blood vessel when a high frequency current is supplied under the same condition to seal the small diameter blood vessel and the large diameter blood vessel. The horizontal axis shows time t during which sealing treatment is being performed.

As shown in Fig. 6A, the impedances Z 1 and Z2 greatly differ from each other in variation, and therefore the method in the prior art of detecting an impedance value, stopping the output when the value reaches a preset threshold and ending the sealing treatment is limited to cases in a narrow range of blood vessel diameter.

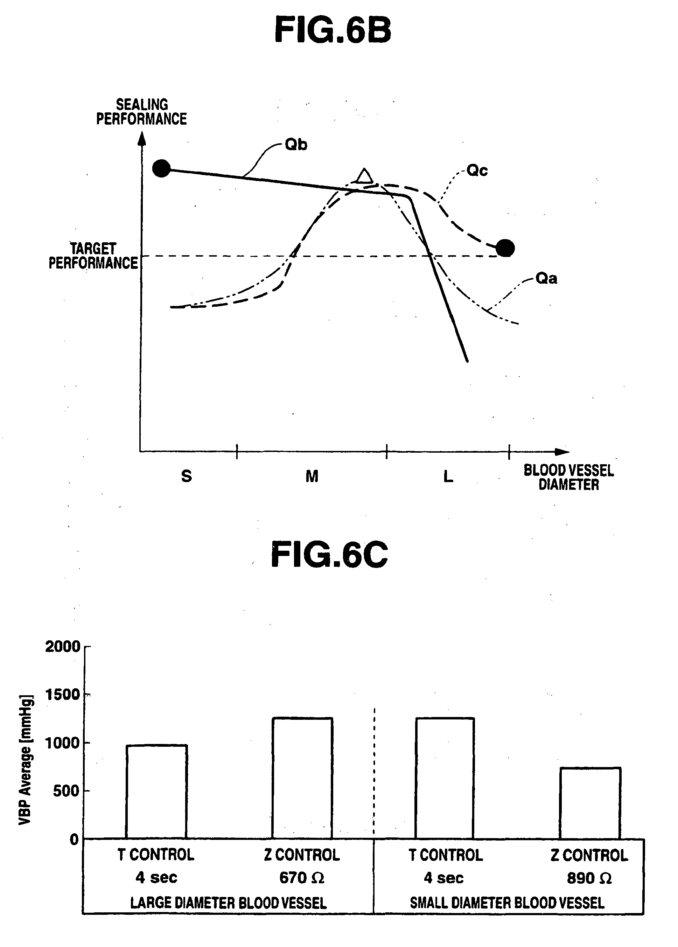

A characteristic Qa shown by a two-dot dashed line in Fig. 6B schematically illustrates sealing performance when the diameter of blood vessel is changed when a threshold (A) of impedance is set as a control parameter in the case with a medium diameter blood vessel (M) so as to obtain sealing performance that exceeds target performance.

The characteristic Qa results in sealing performance lower than required target performance in the cases of small diameter blood vessel (S) and large diameter blood vessel (L).

Thus, the present embodiment uses the threshold Tm of the output time in addition to the threshold Zs of impedance as a control parameter. As shown in Fig. 6A, the threshold Zs of impedance is set for a large diameter blood vessel so as to obtain appropriate sealing performance. This threshold Zs of impedance may be approximated to be substantially made up of a resistance component only.

In the case of the small diameter blood vessel as shown in Fig. 6A, the threshold Tm of the output time is set so as to be able to secure required sealing performance. The present embodiment performs output control so as to end the sealing treatment when conditions for both thresholds Tm and Zs are satisfied.

An overview of sealing performance in this case is as shown by a solid line and a thick dotted line in Fig. 6B. A characteristic Qb shown by the solid line in Fig. 6B is a characteristic that the threshold Tm of the output time is adjusted (tuned) so as to obtain appropriate sealing performance for a small diameter blood vessel (S).

Furthermore, a characteristic Qc shown by a thick dotted line is a characteristic that the threshold Zs of impedance is tuned for a large diameter blood vessel (L). By performing output control so as to satisfy both thresholds Tm and Zs, sealing performance that exceeds target performance can be achieved as shown in Fig. 6B. To be more specific, output control is performed mainly with the characteristic Qb in the case of a small diameter blood vessel, while output control is performed with the characteristic Qc on the large diameter blood vessel side.

A case has been described in Fig. 6B where tuning of output time is performed for a small diameter blood vessel and tuning of impedance is performed for a large diameter blood vessel. Fig. 6C illustrates measured data showing grounds when such tuning is performed.

Two bars on the left and two bars on the right in Fig. 6C illustrate average blood vessel sealing pressure values (VBP) [mmHg] when sealing treatment is applied using a threshold of output time (4 seconds in a specific example) and a threshold of impedance (where Zs' is 670Ω 890Ω) as control parameters in the cases of a large diameter blood vessel and a small diameter blood vessel respectively.

The blood vessel withstand pressure value is a measured value of a pressure when a blood vessel sealed part which is the blood vessel 17 subjected to sealing (treatment) is burst by applying a water pressure thereto in order to objectively evaluate the sealing strength. Since a standard blood pressure of human being is 120 mmHg, sealing performance is considered sufficient when it is possible to obtain a blood vessel withstand pressure value three times that blood pressure, that is 360 mmHg or more.

Furthermore, in Fig. 6C, output time control is described as "T control" in abbreviated form and impedance control is described as "Z control" in abbreviated form. Furthermore, the measured data in Fig. 6C is an example where the threshold Zs' of impedance is used as a control parameter when an impedance component of a cable such as the high frequency probe 4 for a blood vessel as a living tissue is included, but using the threshold Zs of impedance for only the blood vessel produces a similar result. The measured data is actually obtained according to a high frequency surgery control method of a second embodiment.

In the case of the large diameter blood vessel, it is obvious from the measured data that impedance control is more effective than output time control.

On the other hand, in the case of the small diameter blood vessel, it is obvious that output time control is more effective than impedance control.

Thus, as described in Fig. 6B, the present embodiment performs tuning using the output time in the case of the small diameter blood vessel and performs tuning using impedance in the case of the large diameter blood vessel.

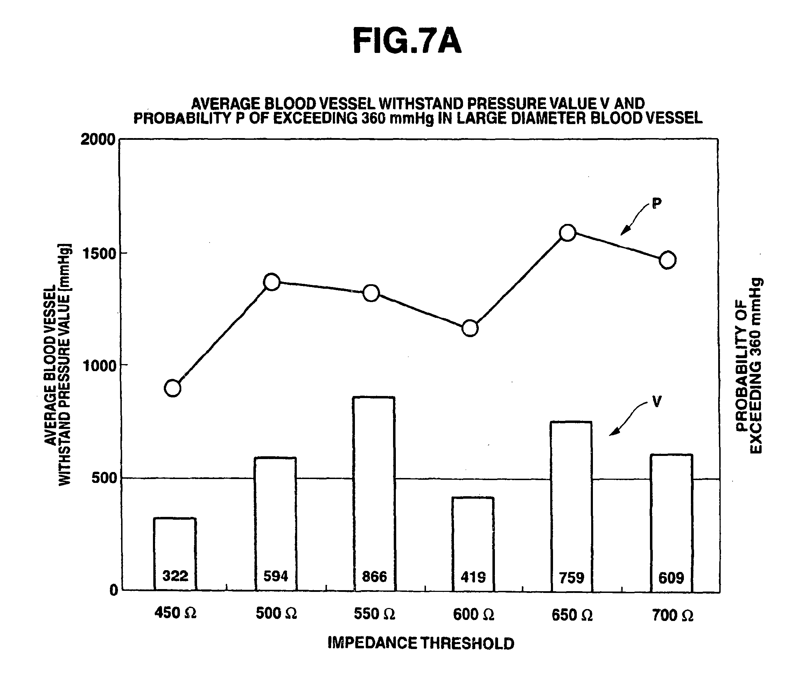

Furthermore, Fig. 7A illustrates measured data of an average blood vessel withstand pressure value V for determining the threshold Zs' of impedance and a probability P exceeding 360 mmHg when tuning is performed for the large diameter blood vessel. That is, Fig. 7A illustrates measured data obtained when the impedance control described in Fig. 6C is performed by changing the threshold Zs' of impedance.

It is obvious from the measured data in Fig. 7A that the threshold Zs' of impedance may be set in the vicinity of, for example, 650Ω with consideration given to the fact that the probability P exceeding 360 mmHg shown by a polygonal line of is high.

That is, the threshold Zs' of impedance as a tuning value of impedance is 650Ω and the threshold Zs of net impedance of the blood vessel 17 portion in this case is 925Ω. Therefore, the vicinity of 700Ω to 1100Ω including this value 925Ω may be set to the threshold Zs of impedance of the blood vessel 17 as the living tissue to be treated (to be operated on).

The probability P that exceeds 360 mmHg in Fig. 7A shows a relative value which is a probability of exceeding 360 mmHg statistically calculated from the blood vessel withstand pressure value obtained.

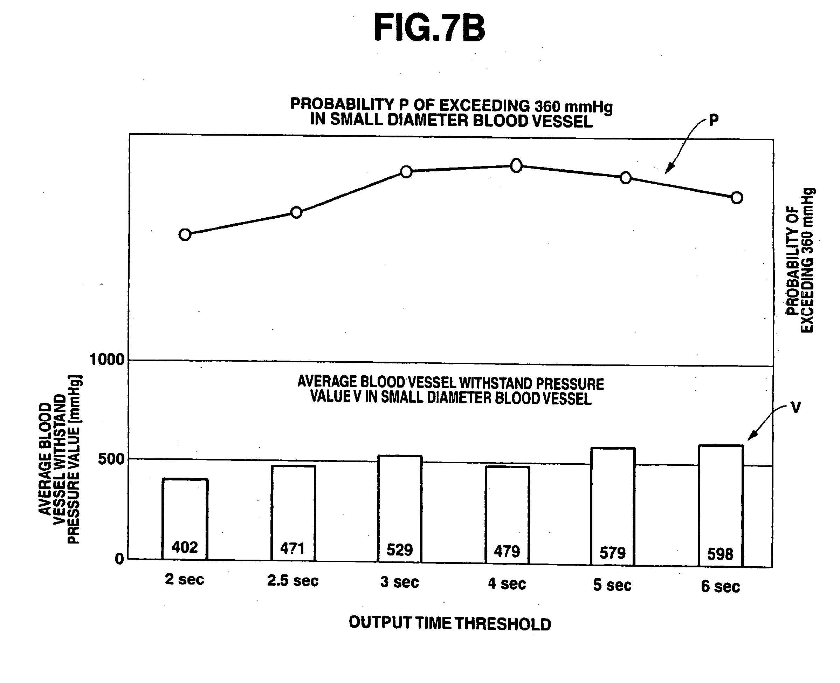

Furthermore, Fig. 7B illustrates measured data of an average blood vessel withstand pressure value V for determining the threshold Tm of the output time Ta and the probability P exceeding 360 mmHg when tuning is performed for the small diameter blood vessel. That is, Fig. 7B illustrates measured data obtained when the output time control described in Fig. 6C is performed by changing the threshold Tm of the output time Ta. The upper part in Fig. 7B shows measured data of the probability P exceeding 360 mmHg and the lower part shows the average blood vessel withstand pressure value V.

From the measured data in Fig. 7B, for example, the vicinity of 3 seconds to 6 seconds may be set as the threshold Tm of the output time Ta.

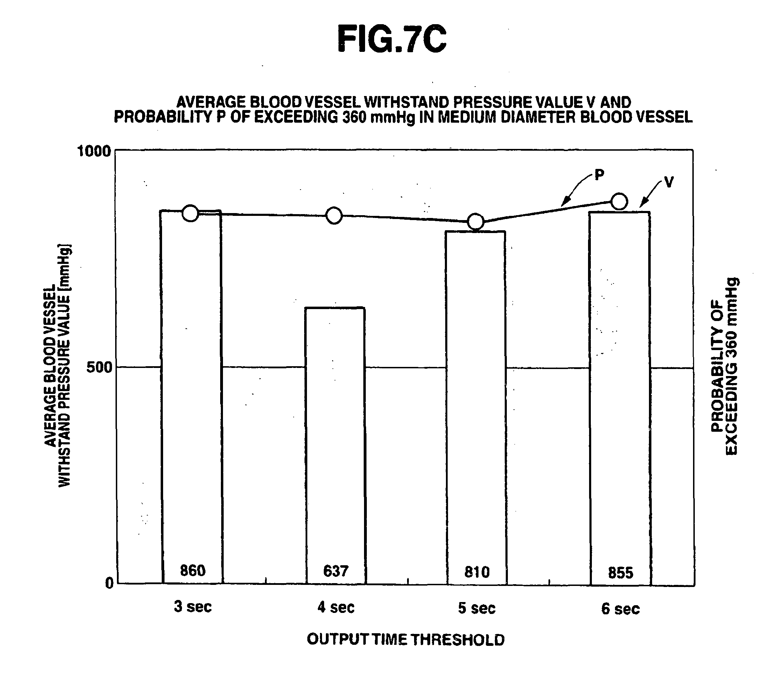

Furthermore, Fig. 7C illustrates measured data of the average blood vessel withstand pressure value V for determining the threshold Tm of output time Ta and the probability P exceeding 360 mmHg when tuning is performed for a medium diameter blood vessel. That is, Fig. 7C illustrates measured data obtained when the output time control described in Fig. 6C is performed by changing the threshold Tm of the output time Ta.

In the measured data in Fig. 7C, although the average blood vessel withstand pressure value V in the case of 4 seconds is somewhat low, since a value nearly twice 360 mmHg is maintained in this case too, any value in the vicinity of, for example, 3 seconds to 6 seconds may be adopted as the threshold Tm of the output time Ta.

Using two control parameters set in this way, it is possible to smoothly perform sealing treatment in the case of any blood vessel 17 of small to large diameter according to the present embodiment as described above. Furthermore, according to the present embodiment, it is possible to perform sealing treatment simply and in a short time in the case of any blood vessel 17 of small to large diameter and alleviate the burden on the operator and patient.

### (Second embodiment)

Next, a second embodiment of the present invention will be described. The configuration of the present embodiment is a configuration similar to that of the first embodiment shown in Fig. 1 and Fig. 2.

The CPU 38a of the control section 38 according to the present embodiment performs output control different from that of the first embodiment. In the first embodiment, sealing treatment is performed in one output mode.

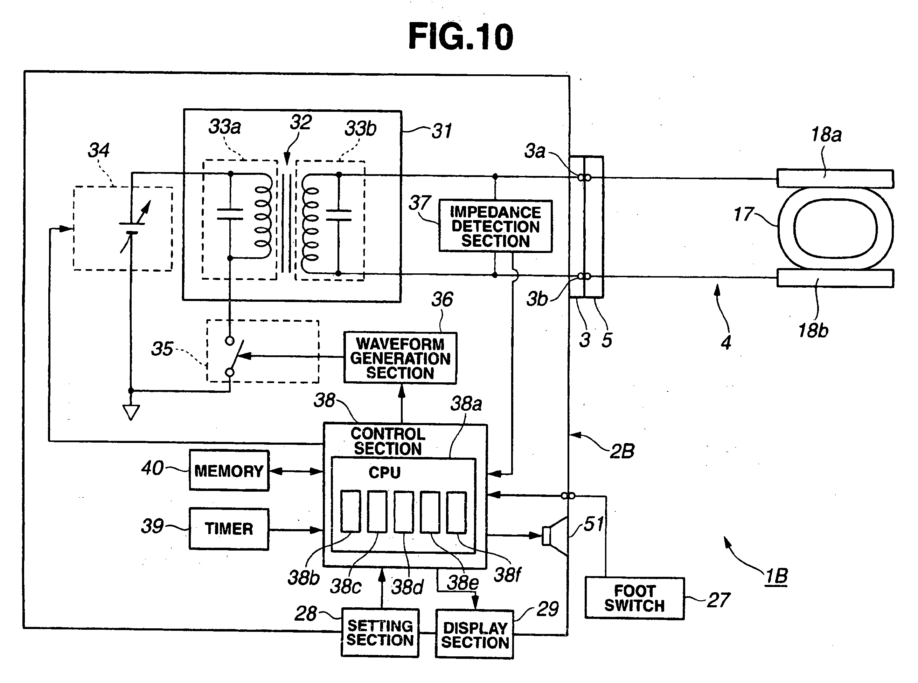

By contrast, in the present embodiment, the CPU 38a performs control so as to use the intermittent output mode when starting the output and switch the mode from the intermittent output mode to the continuous output mode when the detected impedance Za reaches a third threshold Zf of impedance as a control parameter used to switch a preset output mode. That is, in the present embodiment, the CPU 38a has a function of a switching control section (indicated by 38d in Fig. 10 which will be described later) that performs switching control of the output mode. The threshold Zf is a value by far smaller than the threshold Zs, to be more specific, on the order of 101Ω. The threshold Zf is stored in the memory 40 (see Fig. 2).

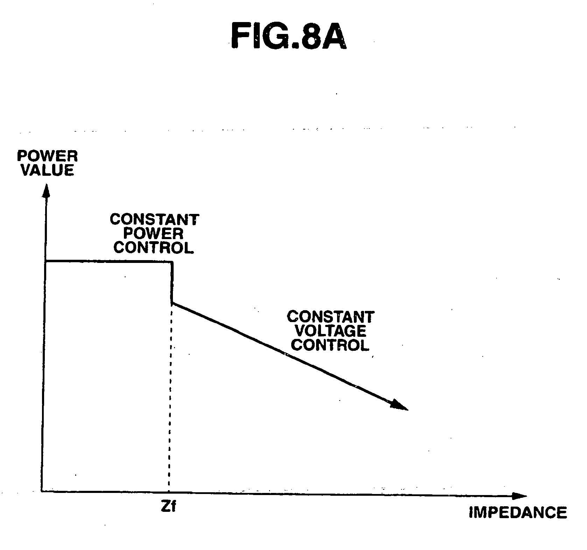

As shown in Fig. 8A, the present embodiment performs constant power control for the period in the intermittent output mode and performs constant voltage control after reaching the threshold Zf of impedance and shifting to the continuous output mode. When the constant power control is shifted to the constant voltage control, the amount of high frequency energy injected into the blood vessel 17 is gradually reduced.

By switching between the output modes in this way, the present embodiment allows sealing treatment to be smoothly performed for any blood vessel of small to large diameter. In Fig. 8A, the horizontal axis shows an impedance and the vertical axis shows a power value.

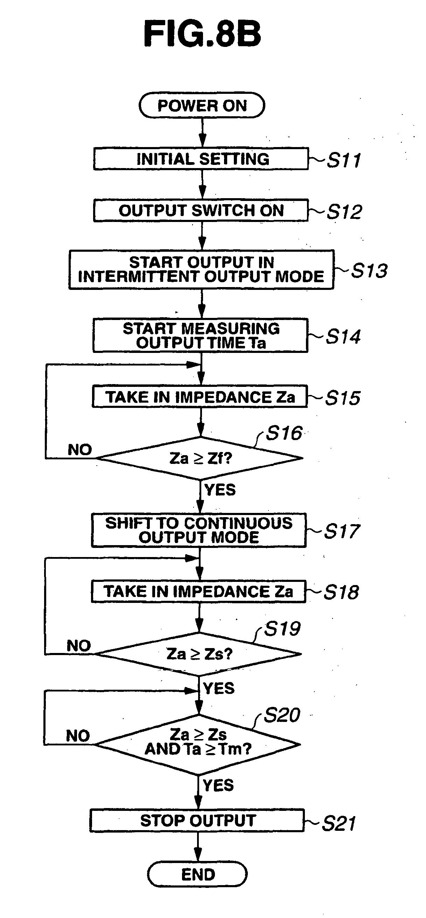

Next, a high frequency surgery control method according to the present embodiment will be described with reference to Fig. 8B. After turning ON the power, the operator makes an initial setting in first step S 11.

In the present embodiment, the threshold Tm of the output time and the threshold Zs of impedance as control parameters are set to 4 seconds and 925Ω respectively by default. Furthermore, the threshold Zf of impedance used for switching between output modes is set to 101Ω by default.

Furthermore, the intermittent output mode period is set by default such that a high frequency current is outputted in a cycle including 60 ms of ON and 30 ms of OFF with constant power of 40 W. Furthermore, the continuous output mode period is set by default such that a high frequency current is outputted at a constant voltage of 70 Vrms.

Therefore, when performing sealing treatment with the default setting as is, the operator can perform the treatment without changing these values. The operator may also operate the setting section 28 to make a selective setting from, for example, 3 seconds of level 1, 4 seconds of level 2 and 5 seconds of level 3, which are prepared in advance, as the threshold Tm of the output time.

The operator grasps the blood vessel to be treated using the electrodes 18a and 18b at the distal end of the high frequency probe 4 and turns ON the foot switch 27 as the output switch as shown in step S12. The CPU 38a of the control section 38 then performs control so as to cause the high frequency current generation section 31 to generate a high frequency current.

As shown in step S 13, the high frequency power supply apparatus 2 outputs a high frequency current from the output end in the intermittent output mode. The high frequency current is transmitted to the blood vessel 17 via the high frequency probe 4, the high frequency current passes through the blood vessel 17 and sealing treatment is started. That is, the output starts in the intermittent output mode.

In this case, as shown in step S 14, the CPU 38a causes the timer 39 to start measuring (counting) the output time Ta of the high frequency current.

Furthermore, as shown in step S15, the CPU 38a takes in a detected impedance Za in a predetermined cycle using the impedance detection section 37.

As shown in next step S16, the CPU 38a judges whether or not the impedance Za taken in has reached a preset threshold Zf (to be more specific, Zf=101Ω), that is, Za≥Zf.

When the condition of Za≥Zf is not satisfied (that is, Za<Zf), the CPU 38a returns to the processing in step S 15.

On the other hand, when the judgment result shows that the condition of Za≥Zf is satisfied, the CPU 38a moves to processing in step S17. In step S17, the CPU 38a switches (shifts) the high frequency current of the high frequency current generation section 31 from the intermittent output mode to the continuous output mode. Therefore, the high frequency current in the continuous output mode flows through the blood vessel 17.

Furthermore, in next step S18, the CPU 38a takes in the detected (measured) impedance Za from the impedance detection section 37 in a predetermined cycle.

As shown in next step S19, the CPU 38a judges whether or not the impedance Za taken in has reached the preset threshold Zs (to be more specific, Zs=925Ω), that is, Za≥Zs.

When the condition of Za≥Zs is not satisfied (that is, Za<Zs), the CPU 38a returns to the processing in step S 18.

On the other hand, when the judgment result shows that the condition of Za≥Zs is satisfied, the CPU 38a moves to processing in step S20. In step S20, the CPU 38a judges whether or not the measured (counted) output time Ta has reached the threshold Tm, that is, Ta≥Tm from the timer 39. Since the judgment result in step S 19 before the judgment in step S20 shows that the condition of Za≥Zs is satisfied, it is substantially judged in step S20 whether or not Za≥Zs and Ta≥Tm.

When the judgment result in step S20 shows that Ta≥Tm is not satisfied (that is, Ta<Tm), the CPU 38a returns to the processing in step S20. On the other hand, when the judgment result shows that the condition of Ta≥Tm is satisfied, the CPU 38a moves to processing in step S21. In step S21, the CPU 38a performs control so as to stop the output. The CPU 38a then ends the control processing on the sealing treatment in Fig. 8B.

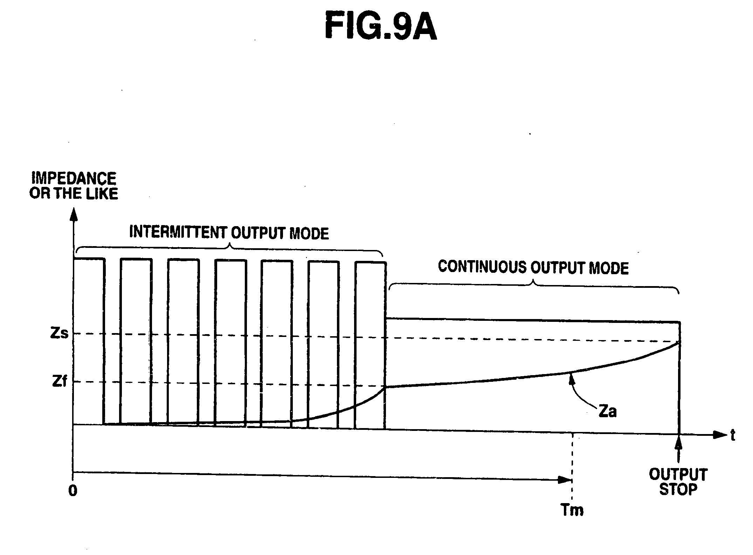

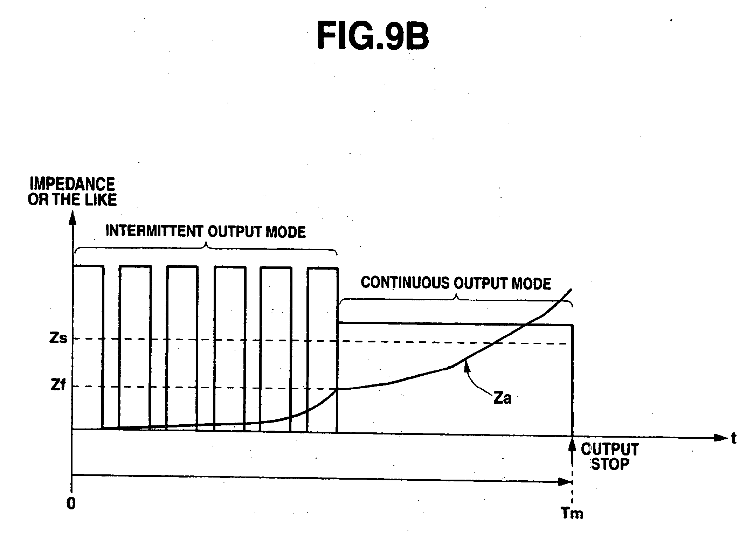

Fig. 9A and Fig. 9B illustrate a variation of the impedance Za when the high frequency control method in Fig. 8B is applied to a large diameter blood vessel and a small diameter blood vessel.

As is clear from a comparison of Fig. 9A and Fig. 9B, since the impedance Za increases more slowly in the case of the large diameter blood vessel than in the case of the small diameter blood vessel, the time until the impedance Za reaches the threshold Zf is longer than in the case of the small diameter blood vessel. Therefore, in the case of the large diameter blood vessel, the treatment time in the intermittent output mode is longer than in the case of the small diameter blood vessel.

When the impedance Za reaches the threshold Zf, the output mode shifts to the continuous output mode. After the shift, even when the output time Ta reaches the threshold Tm of the output time, the impedance Za in the case of the large diameter blood vessel is less than the threshold Zs. Furthermore, when the continuous output mode continues and the impedance Za thereof reaches or exceeds the threshold Zs, the output is stopped.

On the other hand, in the case of the small diameter blood vessel, the impedance Za increases sooner than in the case of the large diameter blood vessel, and therefore the impedance Za reaches the threshold Zf in a shorter time than in the case of the large diameter blood vessel.

When the impedance Za reaches the threshold Zf, the output mode shifts to the continuous output mode. After the shift, before the output time Ta reaches the threshold Tm of the output time, the impedance Za thereof exceeds the threshold Zs. Furthermore, the continuous output mode continues and when the output time Ta reaches or exceeds the threshold Tm, the output is stopped.

The present embodiment allows sealing treatment to be smoothly performed such that a sufficient blood vessel withstand pressure value is obtained for any blood vessel 17 of small to large diameter.

In the case of the small diameter blood vessel, the aforementioned threshold Tm of output time is a value on the lower limit side of the time set so as to satisfy a target value of the blood vessel withstand pressure value required by sealing treatment and sealing treatment may be performed for a longer time than the threshold Tm in the case of the small diameter blood vessel.

Furthermore, in the case of the large diameter blood vessel, the impedance Za is smaller than the threshold Zs of impedance during the output time until the threshold Tm, and therefore the value of the threshold Tm may also be set to a value slightly greater than 3 to 6 seconds (on the order of 1 second).

### (Third embodiment)

Next, a third embodiment of the present invention will be described. The configuration of the present embodiment is a configuration similar to that of the first embodiment shown in Fig. 1 and Fig. 2. Fig. 10 illustrates a configuration of a high frequency power supply apparatus 2B in a high frequency surgery apparatus 1B of the present embodiment.

In the high frequency power supply apparatus 2B, the CPU 38a making up the control section 38 in the high frequency power supply apparatus 2 in Fig. 2 includes an impedance variation calculation section 38e that calculates an impedance variation ΔZa per predetermined time from an impedance Za detected by the impedance detection section 37. Furthermore, the CPU 38a includes a judging section that judges whether or not the calculated impedance variation ΔZa is equal to or above a preset threshold ΔZt.

Furthermore, upon judging that the calculated impedance variation ΔZa is equal to or above the preset threshold ΔZt, the CPU 38a has a function of a second output control section 38f that performs output control so as to reduce a high frequency current (or high frequency energy) that performs sealing treatment. The output control section 38c may include this function as well.

In other words, the CPU 38a performs output control so that the calculated impedance variation ΔZa falls within a predetermined range.

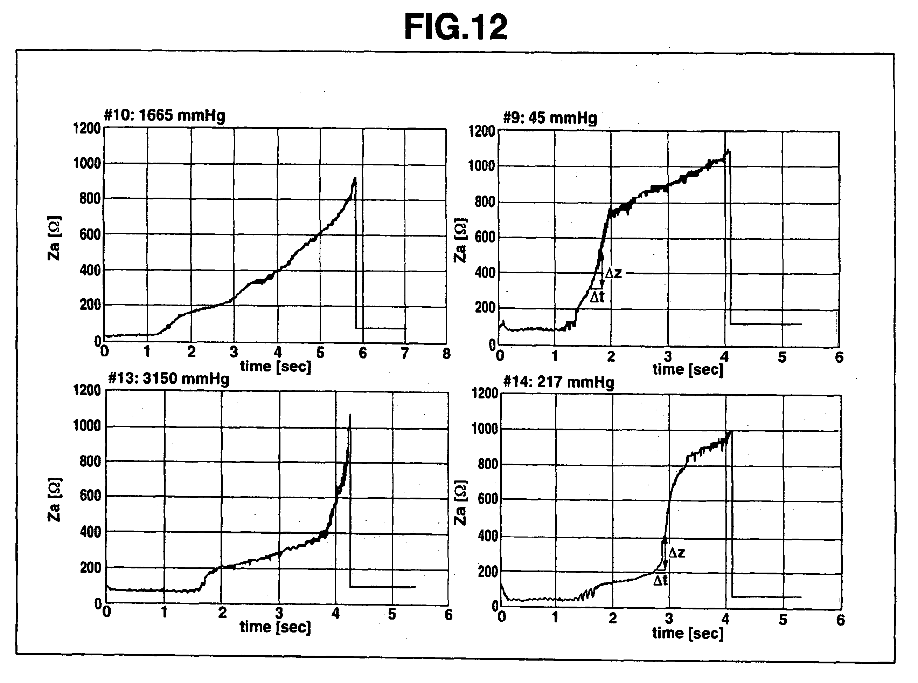

When calculating the impedance variation ΔZa, the value of the predetermined time is set to, for example, on the order of several tens of ms to 100 ms. Furthermore, the threshold ΔZt is set to a value on the order of 200Ω/200 ms (=1kΩ/s) or slightly smaller than this value. The threshold ΔZt is set based on measured data shown in Fig. 12 which will be described later.

The CPU 38a also has the function of the switching control section 38d described in the second embodiment.

Therefore, the present embodiment corresponds to the second embodiment further provided with the impedance variation calculation section 38e and the second output control section 38f.

The second output control section 38f reduces a set value of high frequency power during a period in an intermittent output mode and reduces a set value of voltage during a period in a continuous output mode.

The high frequency power supply apparatus 2B of the present embodiment includes a notifying section 51 that notifies the operator et al., when sealing treatment is performed using control parameters, that the output is not stopped even after a lapse of an allowable output time.

To be more specific, when a threshold Tm of an output time Ta has elapsed, the CPU 3 8a judges whether or not a threshold Te set to a value greater than the threshold Tm (e.g., 10 seconds) is exceeded. When the threshold Te is exceeded, the operator is vocally notified through, for example, a speaker that makes up the notifying section 51 that a standard treatment time has been exceeded.

Notification is not limited to notification by voice but may also be realized by means of display on a display section 29. After the notification, stoppage of the output may be realized interlocked therewith. Furthermore, the operator may be asked to judge whether or not to stop the output and the stoppage or continuation of the output may be decided according to the judgment result.

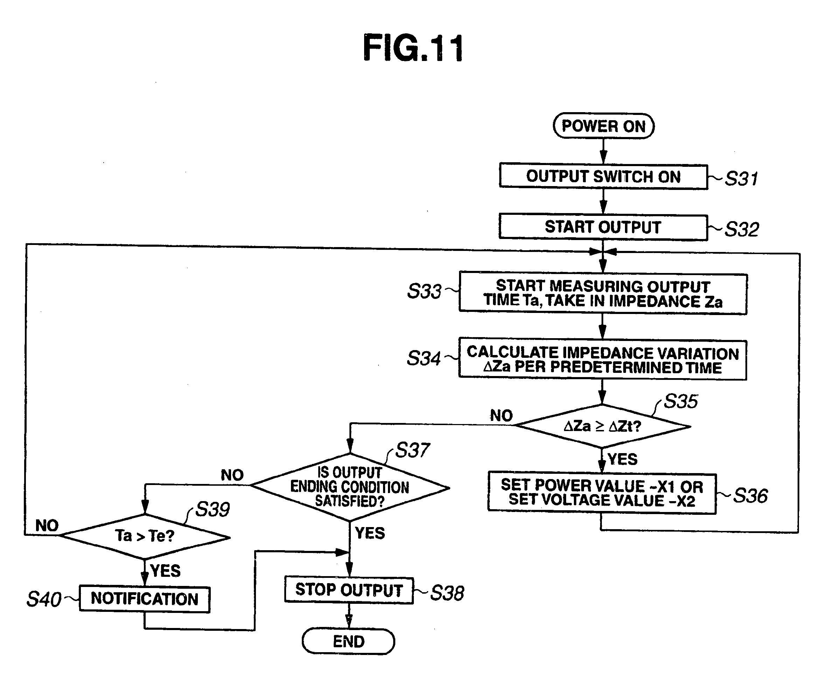

The rest of the configuration is similar to the configuration of the second embodiment. The processing procedure for output control of the present embodiment corresponding to a case where sealing treatment according to the second embodiment is performed is as shown in Fig. 11.

When the power is turned ON, the high frequency surgery apparatus 1B is set in an operating state. When the operator turns ON the output switch as in step S31, a high frequency current is supplied to a blood vessel to be treated through the high frequency probe 4 as shown in step S32 and the output is started. As shown in step S33, the CPU 38a causes the timer 39 to start to measure an output time Ta and causes the impedance detection section 37 to take in the detected impedance Za.

Furthermore, in next step S34, the CPU 38a calculates an impedance variation ΔZa per predetermined time. The predetermined time may also be set to an appropriate time.

In next step S35, the CPU 38a judges whether or not the impedance variation ΔZa reaches or exceeds a preset threshold ΔZt. That is, the CPU 38a judges whether or not ΔZa≥ΔZt.

When this judgment condition is satisfied, in next step S36, the CPU 38a reduces the output by lowering the set power value by a value of X1 or lowering the set voltage value by X2, and then returns to the processing in step S33.

When the output is started as described in the second embodiment, treatment is performed in an intermittent output mode with constant power. Therefore, when the judgment condition in step S35 is met during the period in the intermittent output mode, the set power value is reduced by X1. When, for example, the set power value is 40W, the set power value is reduced by on the order of several W. When the judgment condition in step S35 is met during the period in the continuous output mode, the set voltage value is reduced by X2. When, for example, the set voltage value is 70 Vrms, the set voltage value is reduced by on the order of 5 Vrms.

On the other hand, when the judgment condition in step S35 is not satisfied, the CPU 38a moves to step S37 and in step S37, the CPU 38a judges whether or not the output ending condition is satisfied. To be more specific, the output ending condition is the judgment processing in step S20 in Fig. 8B. When the output ending condition is satisfied, in step S38, the CPU 38a performs processing of stopping the output and ends the output control in Fig. 11.

In the case of a judgment result that the output ending condition in step S37 is not satisfied, the CPU 38a moves to processing in step S39 and in this step S39, the CPU 38a judges whether or not the output time Ta exceeds a threshold Te close to a maximum value allowable as a preset standard output time. That is, the CPU 38a judges whether or not Ta>Te.

When the judgment condition is not satisfied, the CPU 38a returns to step S33 and repeats the aforementioned processing. On the other hand, when the judgment condition in step S39 is satisfied, in next step S40, the CPU 38a notifies through the notifying section 51 that the standard output time (treatment time) is exceeded and then moves to processing in step S38.

By performing output control as shown in Fig. 11, it is possible to reduce the possibility that treatment may be performed departing from the characteristics of the standard impedance Za according to the second embodiment shown in Fig. 9A and Fig. 9B.

Fig. 12 illustrates impedance variations in cases with near-best blood vessel withstand pressure values in a plurality of samples sealed according to the second embodiment (samples #10 and #13 on the left) and near-minimum blood vessel withstand pressure values (samples #9 and #14 on the right).

In the sample with the near-minimum blood vessel withstand pressure values compared with the near-best sample, a steep impedance variation has occurred until about the middle of the output time (for a lapse of time). A steep impedance variation (ΔZ/Δt), to be more specific, ΔZ/Δt≈200Ω/200 ms has occurred, for example, in the vicinity of 1.5 to 2 seconds in sample #9 and in the vicinity before 3 seconds in sample #14. Thus, the samples showing the occurrence of steep impedance variations (ΔZ/Δt) until about the middle of the output time have shown a tendency that their blood vessel withstand pressure values decrease.

Furthermore, when such samples were examined, a tendency was found that degeneration of the tissue occurred on the surface of the tissue due to an excessive temperature rise, transmission of high frequency energy was blocked by the degeneration of the surface and concrescence effects on the interior of the tissue or dehydrations were often not obtained.

For this reason, the present embodiment performs control to reduce the amount of high frequency energy injected so as to prevent such a steep impedance variation from occurring, resulting in an excessive temperature rise on the surface of the tissue.

To be more specific, when the impedance variation ΔZa exceeds the threshold ΔZt during an intermittent output mode period when a high frequency current is outputted with a constant power value as described above, the constant power value thereof is reduced by a predetermined power value (X1) at a time through a control loop.

On the other hand, when the impedance variation ΔZa exceeds the threshold ΔZt during the period in continuous output mode in which a high frequency current is outputted with a constant voltage value, the constant voltage value thereof is reduced by a predetermined voltage value (X2) at a time through a control loop.

With such output control, the present embodiment not only has effects similar to those of the second embodiment, but also can reduce the probability that an insufficient blood vessel withstand pressure value may be generated when sealing treatment is applied and perform more preferable sealing treatment. The present embodiment may also be applied to the first embodiment.

The present embodiment may reference accumulated past data when sealing treatment is performed, use data such as impedance Za, impedance variation ΔZa or the like at each output time Ta obtained when sealing treatment is actually performed, and estimate sealing strength, to be more specific, an evaluation result of blood vessel withstand pressure values as an objective measure of sealing treatment thereof.

In this case, when known data is not enough to give an evaluation result with predetermined reliability, data may be accumulated until it is possible to give an evaluation result with the predetermined reliability.

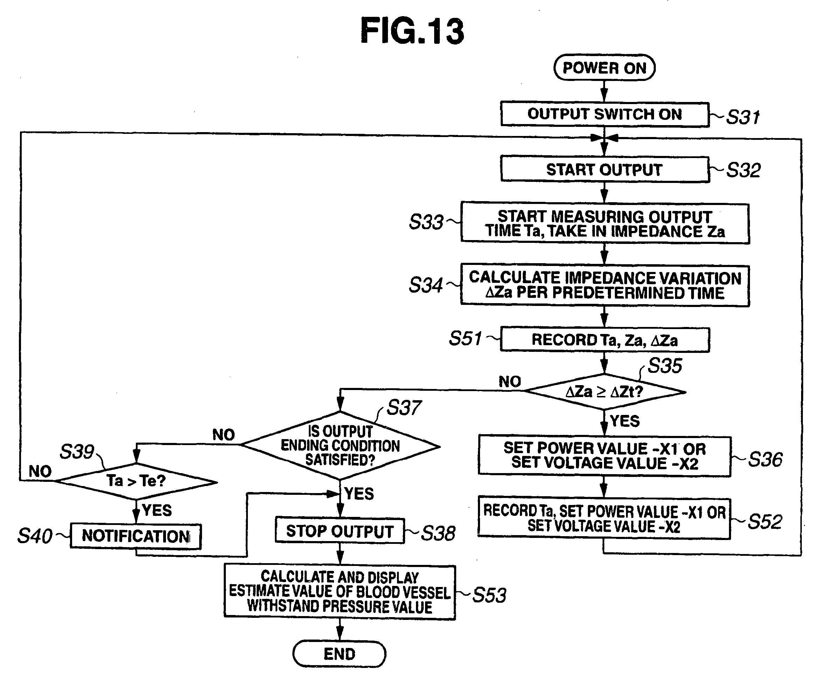

Fig. 13 illustrates a procedure for a high frequency surgery control method designed to notify a blood vessel withstand pressure value as estimated sealing strength after treatment using accumulated data. Since Fig. 13 is only partially different from Fig. 11, only differences will be described.

In step S51 provided between steps S34 and S35 in Fig. 11 in the processing procedure shown in Fig. 13, the CPU 38a records the output time Ta, the impedance Za and the impedance variation ΔZa in recording means such as the memory 40.

Furthermore, in step S52 after step S36, the CPU 38a records the output time Ta, set power value -X1 or set voltage value -X2 in recording means such as the memory 40.

Furthermore, in step S53 after step S38, the CPU 38a calculates an estimate value of blood vessel withstand pressure value estimated in the case of the blood vessel 17 immediately after treatment is ended based on data such as the output time Ta, the impedance Za, the impedance variation ΔZa or the like when sealing treatment is performed in Fig. 13 and the accumulated past data, and displays the estimate value on the display section 29.

For example, the CPU 38a records the accumulated data (however, data whose blood vessel withstand pressure value is known) in the memory 40 or the like with its characteristics such as the value of impedance Za corresponding to the passage of the output time Ta and the impedance variation ΔZa or the like classified into a plurality of patterns.

Furthermore, the CPU 38a records, for example, an average blood vessel withstand pressure value and reliability thereof in the case of the blood vessel 17 subjected to sealing treatment while being included in each pattern in the memory 40 or the like.

The CPU 38a then judges to which pattern of characteristics the data of the blood vessel 17 subjected to sealing treatment corresponds and calculates an estimate value of the blood vessel withstand pressure value in that case. Furthermore, reliability or the like corresponding to the estimate value is also displayed.

By so doing, for the blood vessel 17 treated, the operator can confirm a blood vessel withstand pressure value immediately after the treatment through estimation which can be an objective measure (or guideline) when the blood vessel 17 is sealed.

Furthermore, the blood vessel withstand pressure value through this estimation is assumed to improve reliability as data accumulation advances.

Not only the estimate value of the blood vessel withstand pressure value, but also a judgment result as to whether or not a preset target value (e.g., 360 mmHg) of, for example, the blood vessel withstand pressure value is exceeded and a standard blood vessel withstand pressure value obtained by standard sealing or the like may be displayed or notified together with a value indicating the reliability of the judgment result. In this case, the operator can also confirm an objective judgment result corresponding to the treatment result.

A case has been described in the aforementioned embodiments where the ratio of the ON time to OFF time in the case of, for example, intermittent output is set to 2:1. In this case, the ON time and OFF time may be changed while keeping this ratio according to the type or the like of the high frequency probe 4.

An embodiment configured by partially combining the aforementioned embodiments or the like also belongs to the present invention.

## Claims

1. A high frequency surgery apparatus (1) comprising:
a high frequency current generation section (31) that generates a high frequency current to be transmitted to a living tissue to be operated on;
a high frequency probe (4) that transmits the high frequency current generated to the living tissue and is provided with electrodes (18a, 18b) to perform sealing treatment on the living tissue with the high frequency current;
a time measuring section (39) that measures an output time of the high frequency current of the high frequency current generation section (31);
an impedance detection section (37) that detects an electric impedance of the living tissue;
a memory (40) that stores a first threshold (Tm) and a second threshold (Zs) so as to obtain a desired sealing performance for the living tissue; and
an output control section (38c) that performs control so as to stop the output of the high frequency current upon detecting that the output time measured by the time measuring section (39) exceeds the first threshold (Tm) stored in the memory (40) and detecting that the electric impedance value detected by the impedance detection section (37) exceeds the second threshold (Zs) stored in the memory (40),
**characterized in that**
the first threshold (Tm) is 3 seconds to 6 seconds and the second threshold (Zs) is 700Ω to 1100Ω.

2. The high frequency surgery apparatus (1) according to claim 1, wherein the high frequency current generation section (31) generates the high frequency current in one of two output modes; an intermittent output mode in which the high frequency current is outputted temporally intermittently and a continuous output mode in which the high frequency current is outputted temporally continuously.

3. The high frequency surgery apparatus (1) according to claim 2, wherein the output control section (38c) causes the high frequency current to be outputted in the intermittent output mode when output of the high frequency current is started and causes, upon judging that a value of electric impedance detected by the impedance detection section (37) exceeds a third threshold which is smaller than the second threshold, the high frequency current to be outputted by switching the intermittent output mode to the continuous output mode.

4. The high frequency surgery apparatus (1) according to claim 3, wherein the output control section (38c) performs control so that the ratio of a first period during which the high frequency current is outputted to a second period during which the output of the high frequency current is stopped, the first and second periods forming a cycle in the intermittent output mode, is 2:1.

5. The high frequency surgery apparatus (1) according to claim 4, wherein the first period and the second period are 60 ms and 30 ms respectively.

6. The high frequency surgery apparatus (1) according to claim 3, wherein the output control section (38c) performs control in the intermittent output mode so as to output the high frequency current with a constant power value.

7. The high frequency surgery apparatus (1) according to claim 3, wherein the output control section (38c) performs control in the continuous output mode so as to output the high frequency current with a constant voltage value.

8. The high frequency surgery apparatus (1) according to claim 3, further comprising an impedance variation calculation section (38e) that calculates an impedance variation as a variation of the electric impedance per predetermined time.

9. The high frequency surgery apparatus (1) according to claim 8, wherein the output control section (38c) judges whether or not the impedance variation exceeds a preset fourth threshold and performs control, upon judging that the impedance variation has exceeded the fourth threshold, so as to reduce an output level of the high frequency current.

10. The high frequency surgery apparatus (1) according to claim 9, wherein the output control section (38c) reduces, upon judging that the impedance variation has exceeded the fourth threshold for a period during which the high frequency current is outputted in the intermittent output mode, the set power value of the high frequency current by a predetermined power value.

11. The high frequency surgery apparatus (1) according to claim 9, wherein the output control section (38c) reduces, upon judging that the impedance variation has exceeded the fourth threshold for a period during which the high frequency current is outputted in the continuous output mode, the set voltage value of the high frequency current by a predetermined voltage value.

12. The high frequency surgery apparatus (1) according to claim 9, further comprising a notifying section (51) that notifies, when the output time measured by the time measuring section (39) exceeds a fifth threshold set to a value greater than the first threshold, a user of information that the output time exceeds the fifth threshold.

13. The high frequency surgery apparatus (1) according to claim 8, wherein the treatment with the high frequency current is sealing treatment of a blood vessel as the living tissue and calculates an estimate value of sealing strength corresponding to sealing treatment using data including an electric impedance of the blood vessel during at least a plurality of output times acquired when sealing treatment is performed on the blood vessel based on accumulated data.

## Patentansprüche

1. Chirurgische Hochfrequenzvorrichtung (1) aufweisend:
einen Abschnitt zum Erzeugen eines Hochfrequenzstroms (31), der einen Hochfrequenzstrom zum Übertragen an ein lebendes Gewebe erzeugt, um daran eine Anwendung auszuführen;
eine Hochfrequenzsonde (4), die den erzeugten Hochfrequenzstrom zu dem lebenden Gewebe überträgt und mit Elektroden (18a, 18b) ausgestattet ist, um mit dem Hochfrequenzstrom eine Versiegelungsbehandlung an dem lebenden Gewebe auszuführen;
einen Zeitmessabschnitt (39), der eine Ausgabezeit des Hochfrequenzstroms des Abschnitts zum Erzeugen eines Hochfrequenzstroms (31) misst;
einen Impedanzerfassungsabschnitt (37), der eine elektrische Impedanz des lebenden Gewebes erfasst;
einen Speicher (40), der einen ersten Schwellenwert (Tm) und einen zweiten Schwellenwert (Zs) speichert, um ein gewünschtes Versiegelungsergebnis des lebenden Gewebes zu erlangen; und
einen Aufgabesteuerungsabschnitt (38c), der eine Steuerung ausführt, um die Ausgabe des Hochfrequenzstroms zu stoppen, wenn erfasst wird, dass die durch den Zeitmessabschnitt (39) gemessene Ausgabezeit den in dem Speicher (40) gespeicherten ersten Schwellenwert (Tm) übersteigt und erfasst wird, dass der von dem Impedanzerfassungsabschnitt (37) erfasste elektrische Impedanzwert den in dem Speicher (40) gespeicherten zweiten Schwellenwert (Zs) übersteigt,
**dadurch gekennzeichnet, dass**
der erste Schwellenwert (Tm) drei Sekunden bis sechs Sekunden beträgt und der zweite Schwellenwert (Zs) 700 Ω bis 1100 Ω beträgt

2. Chirurgische Hochfrequenzvorrichtung (1) nach Anspruch 1, bei der der Abschnitt zum Erzeugen eines Hochfrequenzstroms (31) den Hochfrequenzstrom in einem von zwei Ausgabemodi erzeugt: einem Intervallausgabemodus, bei dem der Hochfrequenzstrom in zeitlichen Abständen ausgegeben wird und einem fortlaufenden Ausgabemodus, bei dem der Hochfrequenzstrom zeitlich fortlaufend ausgegeben wird.

3. Chirurgische Hochfrequenzvorrichtung (1) nach Anspruch 2, bei der der Ausgabesteuerungsabschnitt (38c) veranlasst, das der Hochfrequenzstrom in dem Intervallausgabemodus ausgegeben wird, wenn die Ausgabe des Hochfrequenzstroms beginnt, und veranlasst, dass der Hochfrequenzstrom ausgegeben wird durch Schalten von dem Intervallausgabemodus in den fortlaufenden Ausgabemodus, wenn bestimmt wird, dass ein von dem Impedanzerfassungsabschnitt (37) erfasster elektrischer Impedanzwert einen dritten Schwellenwert übersteigt, der kleiner als der zweite Schwellenwert ist.

4. Chirurgische Hochfrequenzvorrichtung (1) nach Anspruch 3, bei der der Ausgabesteuerungsabschnitt (38c) eine Steuerung ausführt, so dass das Verhältnis eines ersten Zeitabschnitts, während dem der Hochfrequenzstrom ausgegeben wird, zu einem zweiten Abschnitt, während dem die Ausgabe des Hochfrequenzstroms gestoppt wird, 2:1 ist, wobei die ersten und zweiten Zeitabschnitte einen Zyklus in dem Intervallausgabemodus bilden.

5. Chirurgische Hochfrequenzvorrichtung (1) nach Anspruch 4, bei der der erste Zeitabschnitt und der zweite Abschnitt 60 ms beziehungsweise 30 ms sind.

6. Chirurgische Hochfrequenzvorrichtung (1) nach Anspruch 3, bei der der Ausgabesteuerungsabschnitt (38c) in dem Intervallausgabemodus eine Steuerung ausführt, so dass der Hochfrequenzstrom mit einem konstanten Leistungswert ausgegeben wird.

7. Chirurgische Hochfrequenzvorrichtung (1) nach Anspruch 3, bei der der Ausgabesteuerungsabschnitt (38c) in dem fortlaufenden Ausgabemodus eine Steuerung ausführt, so dass der Hochfrequenzstrom mit einem konstanten Spannungswert ausgeben wird.

8. Chirurgische Hochfrequenzvorrichtung (1) nach Anspruch 3, ferner aufweisend einen Impedanzveränderungsberechnungsabschnitt (38e), der eine Impedanzveränderung als eine Veränderung der elektrischen Impedanz pro bestimmter Zeit berechnet.

9. Chirurgische Hochfrequenzvorrichtung (1) nach Anspruch 8, bei der der Ausgabesteuerabschnitt (38c) bestimmt, ob die Impedanzveränderung einen voreingestellten vierten Schwellenwert übersteigt oder nicht, und bei Bestimmung, dass die Impedanzveränderung den vierten Schwellenwert überstiegen hat eine Steuerung ausführt, um einen Ausgabewert des Hochfrequenzstroms zu verringern.

10. Chirurgische Hochfrequenzvorrichtung (1) nach Anspruch 9, bei der der Ausgabesteuerungsabschnitt (38c) bei Bestimmung, dass die Impedanzveränderung den vierten Schwellenwert für einen dritten Zeitabschnitt, während dem der Hochfrequenzstrom in dem Intervallausgabemodus ausgegeben wird, überstiegen hat, den eingestellten Leistungswert des Hochfrequenzstroms um einen vorbestimmten Leistungswert verringert.

11. Chirurgische Hochfrequenzvorrichtung (1) nach Anspruch 9, bei der der Ausgabesteuerungsabschnitt (38c) bei Bestimmung, dass die Impedanzveränderung den vierten Schwellenwert für ein Zeitabschnitt überstiegen hat, während dem der Hochfrequenzstrom in dem fortlaufenden Ausgabemodus ausgegeben wird, den eingestellten Spannungswert des Hochfrequenzstroms um einen bestimmten Spannungswert verringert.

12. Chirurgische Hochfrequenzvorrichtung (1) nach Anspruch 9, ferner aufweisend einen Mitteilungsabschnitt (51), der mitteilt, wenn die durch den Zeitmessabschnitt (39) gemessene Ausgabezeit einen fünften Schwellenwert übersteigt, der auf einen Wert größer als der erste Schwellenwert eingestellt ist, dass die Ausgabezeit den fünften Schwellenwert übersteigt.

13. Chirurgische Hochfrequenzvorrichtung (1) nach Anspruch 8, bei der die Behandlung mit dem Hochfrequenzstrom eine Versiegelungsbehandlung eines Blutgefäßes des lebenden Gewebes ist und ein Schätzwert der Versiegelungsstärke entsprechend der Versiegelungsbehandlung berechnet wird unter Verwendung von Daten enthaltend eine elektrische Impedanz des Blutgefäßes während zumindest einer Mehrzahl von Ausgabezeiten, die erlangt wurden, wenn die Versiegelungsbehandlung an den Blutgefäßen ausgeführt wird, basierend auf akkumulierten Daten.

## Revendications

1. Appareil chirurgical (1) haute fréquence comprenant :
une section (31) de génération de courant haute fréquence qui génère un courant haute fréquence destiné à être transmis vers un tissu vivant pour agir sur celui-ci ;
une sonde (4) haute fréquence qui transmet le courant haute fréquence généré vers le tissu vivant et est munie d'électrodes (18a, 18b) pour réaliser un traitement d'obturation sur le tissu vivant avec le courant haute fréquence ;
une section (39) de mesure de temps qui mesure un temps de sortie du courant haute fréquence de la section (31) de génération de courant haute fréquence ;
une section (37) de détection d'impédance qui détecte une impédance électrique du tissu vivant ;
une mémoire (40) qui mémorise un premier seuil (Tm) et un deuxième seuil (Zs) de façon à obtenir une performance d'obturation souhaitée pour le tissu vivant ; et
une section (38c) de commande de sortie qui réalise une commande de façon à arrêter la sortie du courant haute fréquence sur détection que le temps de sortie mesuré par la section (39) de mesure de temps dépasse le premier seuil (Tm) mémorisé dans la mémoire (40), et sur détection que la valeur d'impédance électrique détectée par la section (37) de détection d'impédance dépasse le deuxième seuil (Zs) mémorisé dans la mémoire (40),
**caractérisé en ce que**
le premier seuil (Tm) est de 3 secondes à 6 secondes et le deuxième seuil (Zs) est de 700 Ω à 1100 Ω.

2. Appareil chirurgical (1) haute fréquence selon la revendication 1, dans lequel la section (31) de génération de courant haute fréquence génère le courant haute fréquence dans l'un des deux modes de sortie : un mode de sortie par intermittence dans lequel le courant haute fréquence est délivré en sortie temporairement par intermittence, et un mode de sortie continue dans lequel le courant haute fréquence est délivré en sortie temporairement de manière continue.

3. Appareil chirurgical (1) haute fréquence selon la revendication 2, dans lequel la section (38c) de commande de sortie amène le courant haute fréquence à être délivré en sortie dans le mode de sortie par intermittence lorsque la sortie du courant haute fréquence est lancée et amène, sur jugement qu'une valeur de l'impédance électrique détectée par la section (37) de détection d'impédance dépasse un troisième seuil qui est inférieur au deuxième seuil, le courant haute fréquence à être délivré en sortie en commutant le mode de sortie par intermittence vers le mode de sortie continue.

4. Appareil chirurgical (1) haute fréquence selon la revendication 3, dans lequel la section (38c) de commande de sortie réalise une commande de sorte que le rapport d'une première période pendant laquelle le courant haute fréquence est délivré en sortie par rapport à une deuxième période pendant laquelle la sortie du courant haute fréquence est arrêtée, les première et deuxième périodes formant un cycle dans le mode de sortie par intermittence, est de 2:1.

5. Appareil chirurgical (1) haute fréquence selon la revendication 4, dans lequel la première période et la deuxième période sont de 60 ms et 30 ms respectivement.

6. Appareil chirurgical (1) haute fréquence selon la revendication 3, dans lequel la section (38c) de commande de sortie réalise une commande dans le mode de sortie par intermittence de façon à délivrer en sortie le courant haute fréquence avec une valeur de puissance constante.

7. Appareil chirurgical (1) haute fréquence selon la revendication 3, dans lequel la section (38c) de commande de sortie réalise une commande dans le mode de sortie continue de façon à délivrer en sortie le courant haute fréquence avec une valeur de tension constante.

8. Appareil chirurgical (1) haute fréquence selon la revendication 3, comprenant en outre une section (38e) de calcul de variation d'impédance qui calcule une variation d'impédance en tant que variation de l'impédance électrique par temps prédéterminé.

9. Appareil chirurgical (1) haute fréquence selon la revendication 8, dans lequel la section (38c) de commande de sortie juge si oui ou non la variation d'impédance dépasse un quatrième seuil préétabli et réalise une commande, sur jugement que la variation d'impédance a dépassé le quatrième seuil, de façon à réduire un niveau de sortie du courant haute fréquence.

10. Appareil chirurgical (1) haute fréquence selon la revendication 9, dans lequel la section (38c) de commande de sortie réduit, sur jugement que la variation d'impédance a dépassé le quatrième seuil pendant une période pendant laquelle le courant haute fréquence est délivré en sortie dans le mode de sortie par intermittence, la valeur de puissance établie du courant haute fréquence d'une valeur de puissance prédéterminée.

11. Appareil chirurgical (1) haute fréquence selon la revendication 9, dans lequel la section (38c) de commande de sortie réduit, sur jugement que la variation d'impédance a dépassé le quatrième seuil pendant une période pendant laquelle le courant haute fréquence est délivré en sortie dans le mode de sortie continue, la valeur de tension établie du courant haute fréquence d'une valeur de tension prédéterminée.

12. Appareil chirurgical (1) haute fréquence selon la revendication 9, comprenant en outre une section (51) de notification qui notifie, lorsque le temps de sortie mesuré par la section (39) de mesure de temps dépasse un cinquième seuil établi à une valeur supérieure au premier seuil, un utilisateur avec des informations stipulant que le temps de sortie dépasse le cinquième seuil.

13. Appareil chirurgical (1) haute fréquence selon la revendication 8, dans lequel le traitement avec le courant haute fréquence est un traitement d'obturation d'un vaisseau sanguin en tant que tissu vivant et calcule une valeur d'estimation de résistance d'obturation correspondant au traitement d'obturation en utilisant des données incluant une impédance électrique du vaisseau sanguin pendant au moins une pluralité de temps de sortie obtenus lorsque le traitement d'obturation est réalisé sur le vaisseau sanguin sur la base des données accumulées.
